# EUROPÄISCHE PATENTANMELDUNG

(11) **EP 4 011 895 A1**
(43) Veröffentlichungstag der Anmeldung: **15.06.2022**
(21) Anmeldenummer: 20212755.1
(22) Anmeldetag: 09.12.2020
(51) Int. Cl.: C07F 15/00, C07C 67/38

(54) **PLATIN-KOMPLEXE MIT 1,2-SUBSTITUIERTEN BENZYLBASIERTEN DIPHOSPHINLIGANDEN FÜR DIE KATALYSE DER ALKOXYCARBONYLIERUNG ETHYLENISCH UNGESÄTTIGTER VERBINDUNGEN**

(71) Anmelder: Evonik Operations GmbH, 45128 Essen (DE)
(72) Erfinder: YANG, Ji, Nangang District Heilongjiang Province Harbin City (CN); JACKSTELL, Ralf, 18106 Rostock (DE); BELLER, Matthias, 18211 Ostseebad Nienhagen (DE); FRANKE, Robert, 45772 Marl (DE)
(74) Vertreter: Evonik Patent Association

(57) **Zusammenfassung**

Platin-Komplexe mit 1,2-substituierten benzylbasierten Diphosphinliganden für die Katalyse der Alkoxycarbonylierung ethylenisch ungesättigter Verbindungen.

## Beschreibung

Die vorliegende Erfindung betrifft Platin-Komplexe mit 1,2-substituierten benzylbasierten Diphosphinliganden für die Katalyse der Alkoxycarbonylierung ethylenisch ungesättigter Verbindungen.

Die Alkoxycarbonylierung ethylenisch ungesättigter Verbindungen ist ein Prozess mit steigender Bedeutung. Unter einer Alkoxycarbonylierung versteht man die Umsetzung von ethylenisch ungesättigten Verbindungen, wie Olefinen, mit Kohlenmonoxid und Alkoholen in Gegenwart eines Metalls bzw. eines Metallkomplexes und eines Liganden zu den entsprechenden Estern:

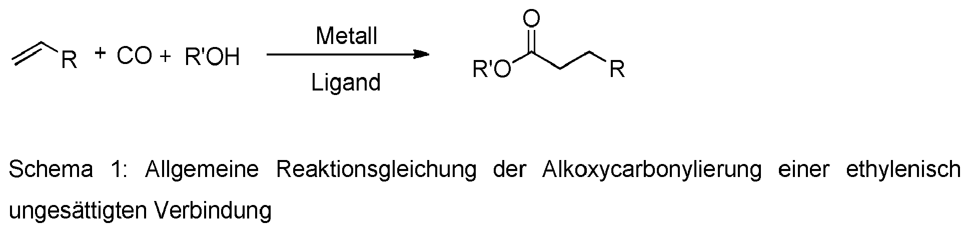

In WO 2011/083305 A1 wird ein Verfahren zur Alkoxycarbonylierung beschrieben. Die dort beschriebenen Komplexe weisen neben den Liganden Palladium als Zentralatom auf.

Ein Nachteil von Palladium ist dessen hoher Preis.

Die technische Aufgabe, welche der vorliegenden Erfindung zugrunde lag, ist die Bereitstellung neuer Komplexe, welche ein Metall als Zentralatom aufweisen, welches einen niedrigeren Preis als Palladium hat. Mit dem Komplex soll zudem bei Alkoxycarbonylierungen gute Umsätze erzielt werden.

Diese Aufgabe wird gelöst durch ein Komplex nach Anspruch 1.

Komplex umfassend Pt und eine Verbindung gemäß Formel (**I**) 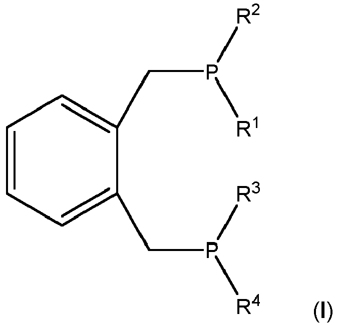 wobei
R¹, R², R³, R⁴ jeweils unabhängig voneinander ausgewählt sind aus -(C₁-C₁₂)-Alkyl, -(C₃-C₁₂)-Cycloalkyl, -(C₃-C₁₂)-Heterocycloalkyl, -(C₆-C₂₀)-Aryl, -(C₆-C₂₀)-Heteroaryl;
mindestens einer der Reste R¹, R², R³, R⁴ für einen -(C₆-C₂₀)-Heteroarylrest mit mindestens sechs Ringatomen steht;
   und
R¹, R², R³, R⁴, falls diese für -(C₁-C₁₂)-Alkyl, -(C₃-C₁₂)-Cycloalkyl, -(C₃-C₁₂)-Heterocycloalkyl,-(C₆-C₂₀-Aryl oder -(C₆-C₂₀)-Heteroaryl stehen,
jeweils unabhängig voneinander mit einem oder mehreren Substituenten ausgewählt aus: - (C₁-C₁₂)-Alkyl, -O-(C₁-C₁₂)-Alkyl, -OH, -NH₂, Halogen substituiert sein können.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Der Begriff (C₃-C₁₂)-Cycloalkyl umfasst mono-, bi- oder tricyclische Kohlenwasserstoffgruppen mit 3 bis 12 Kohlenstoffatomen. Vorzugsweise handelt es sich dabei um (C₅-C₁₂)-Cycloalkyl.

Der Begriff (C₃-C₁₂)-Heterocycloalkyl umfasst nichtaromatische, gesättigte oder teilweise ungesättigte cycloaliphatische Gruppen mit 3 bis 12 Kohlenstoffatomen, wobei eins oder mehrere der Ringkohlenstoffatome durch Heteroatome ersetzt sind. Die (C₃-C₁₂)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen eins oder mehrere der Ringkohlenstoffatome durch Heteroatome oder heteroatomhaltige Gruppen ersetzt. Die Heteroatome oder die heteroatomhaltigen Gruppen sind vorzugsweise ausgewählt unter O, S, N.

Der Begriff (C₆-C₂₀-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Der Begriff (C₆-C₂₀)-Heteroaryl umfasst mono- oder polycyclische aromatische Kohlenwasserstoffreste mit 6 bis 20 Kohlenstoffatomen, wobei eins oder mehrere der Kohlenstoffatome durch Heteroatome ersetzt sind. Bevorzugte Heteroatome sind N, O und S. Die (C₃-C₂₀)-Heteroarylgruppen weisen 6 bis 20, bevorzugt 6 bis 14, besonders bevorzugt 6 bis 10 Ringatome auf. Somit ist beispielsweise Pyridyl im Rahmen dieser Erfindung ein C₆-Heteroarylrest, Furyl ist ein C₅-Heteroarylrest.

Geeignete (C₆-C₂₀)-Heteroarylgruppen mit mindestens sechs Ringatomen sind insbesondere Pyridyl, Pyridazinyl, Pyrimidyl, Pyrazinyl, Benzofuranyl, Indolyl, Isoindolyl.

Der Begriff Halogen umfasst insbesondere Fluor, Chlor, Brom und lod. Besonders bevorzugt sind Fluor und Chlor.

In einer Ausführungsform stehen mindestens zwei der Reste R¹, R², R³, R⁴ für einen -(C₆-C₂₀)-Heteroarylrest mit mindestens sechs Ringatomen.

In einer Ausführungsform stehen die Reste R¹ und R³ jeweils für einen -(C₆-C₂₀)-Heteroarylrest mit mindestens sechs Ringatomen.

In einer Ausführungsform stehen die Reste R¹ und R³ jeweils für 2-Pyridyl.

In einer Ausführungsform stehen R² und R⁴ für -(C₁-C₁₂)-Alkyl.

In einer Ausführungsform stehen R² und R⁴ für *tert*-Butyl.

In einer Ausführungsform weist die Verbindung (**I**) die Struktur (**1**) auf:

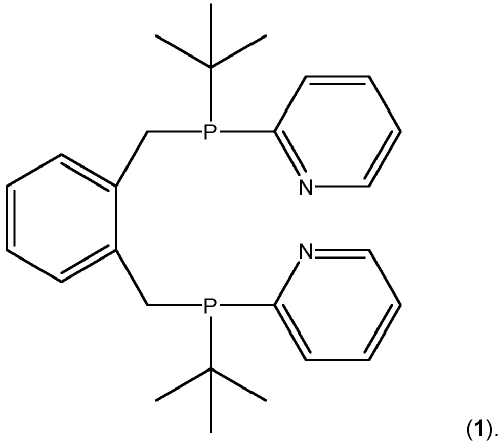

Die Erfindung betrifft weiterhin die Verwendung eines erfindungsgemäßen Komplexes zur Katalyse einer Alkoxycarbonylierungsreaktion.

Verfahren umfassend die Verfahrensschritte:
a) Vorlegen einer ethylenisch ungesättigten Verbindung;
b) Zugabe eines zuvor beschriebenen Komplexes, oder
   eine Verbindung gemäß Formel (I) 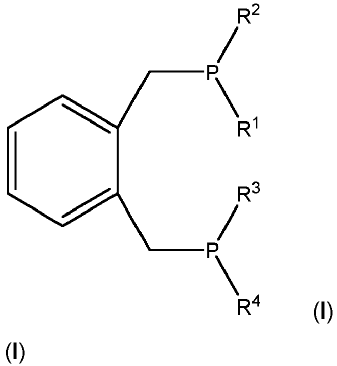 wobei
   R¹, R², R³, R⁴ jeweils unabhängig voneinander ausgewählt sind aus -(C₁-C₁₂)-Alkyl, -(C₃-C₁₂)-Cycloalkyl, -(C₃-C₁₂)-Heterocycloalkyl, -(C₆-C₂₀)-Aryl, -(C₆-C₂₀)-Heteroaryl;
   mindestens einer der Reste R¹, R², R³, R⁴ für einen -(C₆-C₂₀)-Heteroarylrest mit mindestens sechs Ringatomen steht;
      und
   R¹, R², R³, R⁴, falls diese für -(C₁-C₁₂)-Alkyl, -(C₃-C₁₂)-Cycloalkyl, -(C₃-C₁₂)-Heterocycloalkyl,-(C₆-C₂₀-Aryl oder -(C₆-C₂₀)-Heteroaryl stehen,
   jeweils unabhängig voneinander mit einem oder mehreren Substituenten ausgewählt aus: - (C₁-C₁₂)-Alkyl, -O-(C₁-C₁₂)-Alkyl, -OH, -NH₂, Halogen substituiert sein können, und
   einer Substanz, welche Pt umfasst;
c) Zugabe eines Alkohols;
d) Zuführen von CO;
e) Erwärmen des Reaktionsgemisches aus a) bis d), wobei die ethylenisch ungesättigte Verbindung zu einem Ester umgesetzt wird.

Hierbei können die Verfahrensschritte a), b), c) und d) in beliebiger Reihenfolge erfolgen. Üblicherweise erfolgt die Zugabe von CO jedoch, nachdem die Reaktionspartner in den Schritten a) bis c) vorgelegt wurden. Die Schritte d) und e) können gleichzeitig oder nacheinander erfolgen. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

Die in dem erfindungsgemäßen Verfahren als Edukt eingesetzten ethylenisch ungesättigten Verbindungen enthalten eine oder mehrere Kohlenstoff-Kohlenstoff-Doppelbindungen. Diese Verbindungen werden auch im Folgenden zur Vereinfachung als Olefine bezeichnet. Die Doppelbindungen können terminal oder intern sein.

Die ethylenisch ungesättigten Verbindungen können zusätzlich zu der einen oder mehreren Doppelbindungen weitere funktionelle Gruppen enthalten. Dabei umfasst die ethylenisch ungesättigte Verbindung vorzugsweise insgesamt 2 bis 30 Kohlenstoffatome, bevorzugt 2 bis 22 Kohlenstoffatome, besonders bevorzugt 2 bis 12 Kohlenstoffatome.

In einer Variante des Verfahrens umfasst die ethylenisch ungesättigte Verbindung keine weiteren funktionellen Gruppen außer Kohlenstoff-Kohlenstoff-Doppelbindungen.

In einer Variante des Verfahrens ist die ethylenisch ungesättigte Verbindung ausgewählt aus: Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

In einer Variante des Verfahrens ist die ethylenisch ungesättigte Verbindung ausgewählt aus: Propen, 1-Buten, cis-2-Buten, trans-2-Buten, oder Mischungen davon.

In einer Variante des Verfahrens ist die ethylenisch ungesättigte Verbindung ausgewählt aus: 1-Penten, cis-2-Penten, trans-2-Penten, 2-Methyl-1-buten, 2-Methyl-2-buten, 3-Methyl-1-buten, oder Mischungen davon.

Geeignete Gemische ethylenisch ungesättigter Verbindungen sind die sogenannten Raffinate I bis III. Raffinat I umfasst 40 bis 50 % iso-Buten, 20 bis 30 % 1-Buten, 10 bis 20 % cis- und trans-2-Buten, bis zu 1 % 1,3-Butadien und 10 bis 20 % n-Butan und Isobutan. Das Raffinat II ist ein Teil der beim Naphthacracken entstehenden C₄-Fraktion und besteht im Wesentlichen aus den isomeren n-Butenen, Isobutan und n-Butan nach Abtrennung von Isobuten aus Raffinat I. Raffinat III ist ein Teil der beim Naphthacracken entstehenden C₄-Fraktion und besteht im Wesentlichen aus den isomeren n-Butenen und n-Butan.

In einer Variante wird ein Gemisch umfassend iso-Buten, 1-Buten, cis- und trans-2-Buten eingesetzt. Vorzugsweise umfasst das Gemisch 1-Buten, cis- und trans-2-Buten.

In einer Variante des Verfahrens ist der Alkohol in Verfahrensschritt c) ausgewählt aus: Methanol, Ethanol, 1-Propanol, 1-Butanol, 1-Pentanol, 1-Hexanol, 2-Propanol, *tert*-Butanol, 3-Pentanol, Cyclohexanol, Phenol.

In einer Variante des Verfahrens ist der Alkohol in Verfahrensschritt c) Methanol.

In einer Variante des Verfahrens ist die Substanz, welche Pt umfasst ausgewählt aus: Platindichlorid (PtCl₂), Platin(II)-acetylacetonat [Pt(acac)₂], Platin(II)-acetat [Pt(OAc)₂], Dichloro(1,5-cyclooctadiene)platin(II) [Pt(cod)₂Cl₂], Bis(dibenzylideneaceton)platin [Pt(dba)₂], Bis(acetonitrile)dichloropatin(II) [Pt(CH₃CN)₂Cl₂], Platin(cinnamyl)dichlorid [Pt(cinnamyl)Cl₂].

In einer Variante des Verfahrens ist die Substanz, welche Pt umfasst ausgewählt aus: Platindichlorid (PtCl₂), Platin(II)-acetylacetonat [Pt(acac)₂], Platin(II)-acetat [Pt(OAc)₂].

CO wird in Schritt d) vorzugsweise bei einem CO-Partialdruck zwischen 0,1 und 10 MPa (1 bis 100 bar), bevorzugt zwischen 1 und 8 MPa (10 bis 80 bar), besonders bevorzugt zwischen 2 und 6 MPa (20 bis 60 bar) zugeführt.

Die Reaktionsmischung wird in Schritt e) des erfindungsgemäßen Verfahrens vorzugsweise auf eine Temperatur im Bereich von 60 °C bis 160 °C, bevorzugt von 80 °C bis 140 °C, besonders bevorzugt von 100 °C bis 140 °C erwärmt, um die ethylenisch ungesättigte Verbindung zu einem Ester umzusetzen.

Im Folgenden soll die Erfindung anhand eines Ausführungsbeispieles näher erläutert werden.

### Umsetzung von 1-Octen zum Methylester

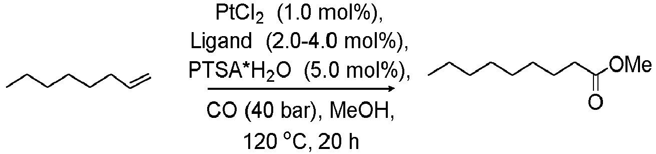

Reaktionsbedingungen: 1-Octen (1.0 mmol), PtCl₂ (0.01 mmol, 1.0 mol%), Ligand: monodentater Phosphinligand (0.04 mmol, 4.0 mol%), bidentater Phosphinligand (0.02 mmol, 2.0 mol%), PTSA·H₂O (Monohydrat der p-Toluolsulfonsäure) (5.0 mol%), MeOH (2.0 mL), Druck(CO): 40 bar, Temperatur: 120 °C, Reaktionszeit: 20 h.

Die Reaktion wurde mit den folgenden Liganden durchgeführt:
Monodentate Phosphinliganden: 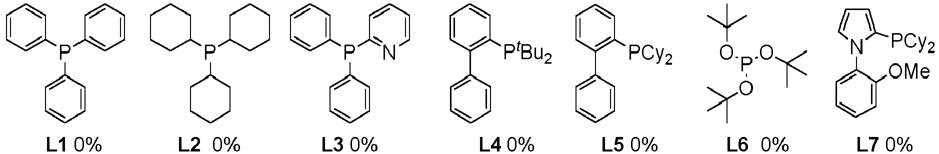
Bidentate Phosphinliganden:

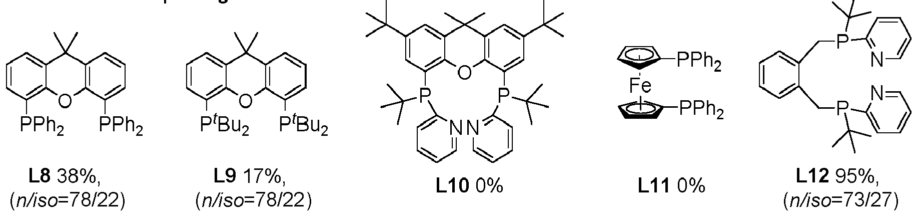

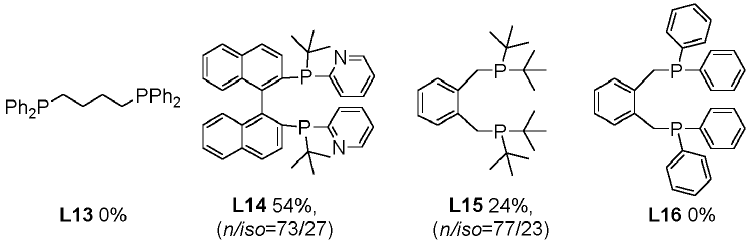

Die jeweilige Ausbeute und n/iso-Selektivität sind unterhalb des Liganden angegeben. Selektivitäten und Ausbeuten wurden gaschromatographisch mit Mesitylen als internem Standard bestimmt.

Von den 16 verwendeten Liganden lieferten 11 keinen Umsatz (0%). Lediglich mit einem Liganden (**L12** = (**1**) ) konnte ein Umsatz von über 60% erzielt werden. Wie die Versuchsreihe zeigt wird der größte Umsatz mit dem erfindungsgemäßen Komplex aus Pt und (1) erzielt.

Der Preis von Pt liegt unter dem von Pd. Die Aufgebe wird somit durch einen erfindungsgemäßen Komplex gelöst.

## Patentansprüche

1. Komplex umfassend Pt und eine Verbindung gemäß Formel (I) 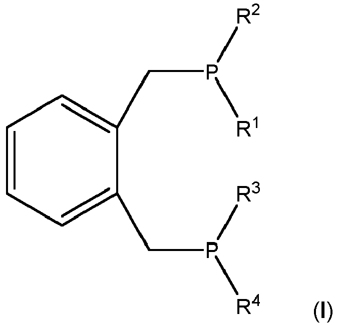 wobei
R¹, R², R³, R⁴ jeweils unabhängig voneinander ausgewählt sind aus -(C₁-C₁₂)-Alkyl,-(C₃-C₁₂)-Cycloalkyl, -(C₃-C₁₂)-Heterocycloalkyl, -(C₆-C₂₀)-Aryl, -(C₆-C₂₀)-Heteroaryl; mindestens einer der Reste R¹, R², R³, R⁴ für einen -(C₆-C₂₀)-Heteroarylrest mit mindestens sechs Ringatomen steht;
und
R¹, R², R³, R⁴, falls diese für -(C₁-C₁₂)-Alkyl, -(C₃-C₁₂)-Cycloalkyl, -(C₃-C₁₂)-Heterocycloalkyl, -(C₆-C₂₀)-Aryl oder -(C₆-C₂₀)-Heteroaryl stehen,
jeweils unabhängig voneinander mit einem oder mehreren Substituenten ausgewählt aus: -(C₁-C₁₂)-Alkyl, -O-(C₁-C₁₂)-Alkyl, -OH, -NH₂, Halogen substituiert sein können.

2. Komplex nach Anspruch 1,
wobei mindestens zwei der Reste R¹, R², R³, R⁴ für einen -(C₆-C₂₀)-Heteroarylrest mit mindestens sechs Ringatomen stehen.

3. Komplex nach einem der Ansprüche 1 bis 2,
wobei die Reste R¹ und R³ jeweils für einen -(C₆-C₂₀)-Heteroarylrest mit mindestens sechs Ringatomen stehen.

4. Komplex nach einem der Ansprüche 1 bis 3,
wobei die Reste R¹ und R³ jeweils für 2-Pyridyl stehen.

5. Komplex nach einem der Ansprüche 1 bis 4,
wobei R² und R⁴ für -(C₁-C₁₂)-Alkyl stehen.

6. Komplex nach einem der Ansprüche 1 bis 5,
wobei R² und R⁴ für *tert*-Butyl stehen.

7. Komplex nach einem der Ansprüche 1 bis 6,
wobei die Verbindung (**I**) die Struktur (**1**) aufweist:

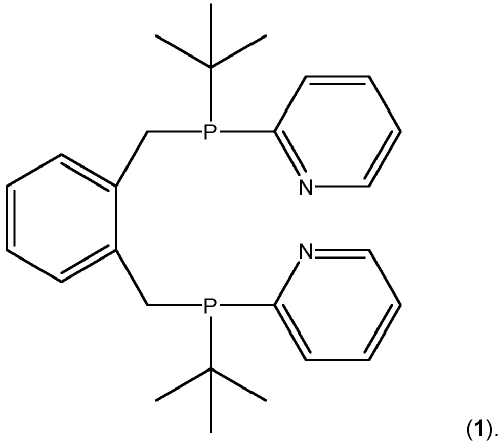

8. Verfahren umfassend die Verfahrensschritte:
a) Vorlegen einer ethylenisch ungesättigten Verbindung;
b) Zugabe eines Komplexes nach einem der Ansprüche 1 bis 7, oder
eine Verbindung gemäß Formel (**I**) 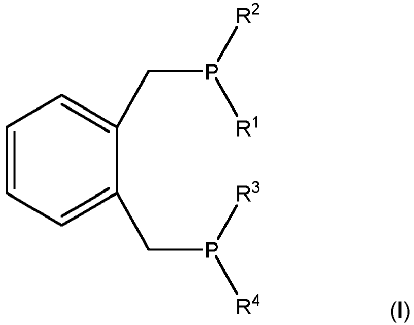 wobei
R¹, R², R³, R⁴ jeweils unabhängig voneinander ausgewählt sind aus -(C₁-C₁₂)-Alkyl,-(C₃-C₁₂)-Cycloalkyl, -(C₃-C₁₂)-Heterocycloalkyl, -(C₆-C₂₀)-Aryl, -(C₆-C₂₀)-Heteroaryl; mindestens einer der Reste R¹, R², R³, R⁴ für einen -(C₆-C₂₀)-Heteroarylrest mit mindestens sechs Ringatomen steht;
und
R¹, R², R³, R⁴, falls diese für -(C₁-C₁₂)-Alkyl, -(C₃-C₁₂)-Cycloalkyl, -(C₃-C₁₂)-Heterocycloalkyl, -(C₆-C₂₀)-Aryl oder -(C₆-C₂₀)-Heteroaryl stehen,
jeweils unabhängig voneinander mit einem oder mehreren Substituenten ausgewählt aus: -(C₁-C₁₂)-Alkyl, -O-(C₁-C₁₂)-Alkyl, -OH, -NH₂, Halogen substituiert sein können, und
einer Substanz, welche Pt umfasst;
c) Zugabe eines Alkohols;
d) Zuführen von CO;
e) Erwärmen des Reaktionsgemisches aus a) bis d), wobei die ethylenisch ungesättigte Verbindung zu einem Ester umgesetzt wird.

9. Verfahren nach Anspruch 8,
wobei die ethylenisch ungesättigte Verbindung ausgewählt ist aus: Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

10. Verfahren nach einem der Ansprüche 8 oder 9,
wobei der Alkohol in Verfahrensschritt c) ausgewählt ist aus: Methanol, Ethanol, 1-Propanol, 1-Butanol, 1-Pentanol, 1-Hexanol, 2-Propanol, *tert*-Butanol, 3-Pentanol, Cyclohexanol, Phenol.

11. Verfahren nach einem der Ansprüche 8 bis 10,
wobei der Alkohol in Verfahrensschritt c) Methanol ist.

12. Verfahren nach einem der Ansprüche 8 bis 11,
wobei die Substanz, welche Pt umfasst ausgewählt ist aus: Platindichlorid (PtCl₂), Platin(II)-acetylacetonat [Pt(acac)₂], Platin(II)-acetat [Pt(OAc)₂], Dichloro(1,5-cyclooctadiene)platin(II) [Pt(cod)₂Cl₂], Bis(dibenzylideneaceton)platin [Pt(dba)₂], Bis(acetonitrile)dichloropatin(II) [Pt(CH₃CN)₂Cl₂], Platin(cinnamyl)dichlorid [Pt(cinnamyl)Cl₂]

13. Verfahren nach einem der Ansprüche 8 bis 12,
wobei die Substanz, welche Pt umfasst ausgewählt ist aus: Platindichlorid (PtCl₂), Platin(II)-acetylacetonat [Pt(acac)₂], Platin(II)-acetat [Pt(OAc)₂].
